# EUROPEAN PATENT SPECIFICATION

(11) **EP 1 827 323 B1**
(45) Date of publication and mention of the grant of the patent: **09.07.2008**
(21) Application number: 05854087.3
(22) Date of filing: 14.12.2005
(51) Int. Cl.: A61F 2/44

(54) **VERTEBRAL COLUMN OSTEOSYNTHESIS DEVICE COMPRISING A CAGE, AN ANTERIOR PLATE AND TWO LONGITUDINAL RODS**
WIRBELSÄULEN-OSTEOSYNTHESE-VORRICHTUNG MIT EINEM KÄFIG, EINER VORDERPLATTE UND ZWEI LÄNGSSTANGEN
DISPOSITIF D'OSTEOSYNTHESE POUR COLONNE VERTEBRALE COMPRENANT UNE CAGE, UNE PLAQUE ANTERIEURE ET DEUX TIGES LONGITUDINALES

(30) Priority: 16.12.2004 FR 0413438
(43) Date of publication of application: 05.09.2007
(73) Proprietor: Warsaw Orthopedic, Inc., Warsaw, IN 46581 (US)
(72) Inventor: PEUL, Wilco, C., NL-2313 EN Leiden (NL); D'AMORE, Jean-Francois, F-42780 Stella Plage (FR); DUPONT, Phillipe, 77410 Claye Souilly (FR)
(74) Representative: Hughes, Andrea Michelle
(86) International application number: PCT/US2005/045301
(87) International publication number: WO 2006/065932

(56) References cited:
- EP-A- 1 188 424
- US-A- 5 092 893
- US-A- 6 159 211
- US-A1- 2004 199 254

## Description

The invention relates to surgery of the vertebral column, particularly in the cervical area.

Various vertebral column osteosynthesis devices are known and used in surgery of the cervical vertebral column when one or several cervical vertebrae have been seriously injured by a tumour or a traumatism, such as a severe fracture, making them very unstable.

Some devices comprise a bone graft enclosed in a cage to fuse two vertebral plates and an anterior plate together. Posterior processes are kept. But they are inevitably unstable.

These devices can be slightly improved to a certain extent by adding a posterior attachment plate on levels superadjacent and subjacent to the lesion. For example, US2004/0199254 proposes an intervertebral arthrodesis device designed to be inserted in an intervertebral space separating the opposite plates of the two adjacent vertebrae, characterised in that it comprises at least one structure called intervertebral cage presenting the shape of a ring. However, when several vertebrae are concerned, there is a large lever arm at the lesion. And once again, the posterior processes at the injured level are not fixed.

Another more radical solution consists of completely removing the injured vertebra(e) and associated disks and replacing them by a solid block in which a bone graft can be placed, making a fusion between the vertebrae retained above and below the injured level. However, anterior and posterior stability is not achieved and an anterior plate and / or a posterior plate has (have) to be added to this device, which was independent from the block in known embodiments. However even under these conditions, stability is not optimal.

The purpose of the invention is to provide surgeons with an osteosynthesis device for the vertebral column to achieve optimal anterior and posterior stability of the vertebral column, particularly the cervical part of the vertebral column, when one or several injured vertebrae have been removed.

To achieve this, the purpose of the invention is a vertebral column osteosynthesis device of the type comprising at least one cage containing a bone graft that will be substituted for at least one vertebra, and the vertebrae are fused with the cage placed between them, and at least one anterior plate thai will be fixed to the said vertebrae between which the said cage or at least one of the said cages is placed, characterised in that the said device also comprises:
- means of attachment of the said anterior plate or at least one of the said anterior plates to the cage facing it;
- two longitudinal rods that will extend along the vertebral column on each side of the processus spinosus, means of attachment of the ends of the said rods to healthy ends of the patient's skeleton, and means of connection of each rod to the said cage or to at least one of the said cages.

The said cage may comprise an annular element at at least one of its ends for stacking two cages or for anchoring the said cage in a vertebral plate.

The said means of connection of each rod to the said cage may be composed of threaded orifices formed in the cage and screws penetrating into the said threaded orifices, the heads of each of the said screws comprising means of reception and locking of one of the said rods.

The said threaded orifices may be formed on posterior edges of the cage and control the orientations of the said screws making them correspond to the orientations of the natural processes of the vertebrae to be replaced by the cage.

The said screws may be inserted into sleeves connected to the said cage.

One of the ends of the said rods may be shaped so that they can be fixed to the patient's occiput.

The device may comprise several cages, and one anterior plate for each cage.

At least one of the said cages may comprise means of connection to the said rods distributed on several of its levels.

The dimensions and shape of the said at least one cage, possibly provided with one or several annular elements, may be compatible with the replacement of a cervical vertebra or several successive cervical vertebrae.

As will have been understood, the invention is based on a combination of:
- an anterior plate fixed at both its ends to conserved vertebrae above and below the level of the injured vertebrae that were removed;
- a cage or a superposition of cages enclosing a bone graft that is substituted for the injured vertebral body or bodies and their associated disks; and the cage is fixed to the anterior plate at at least one of its levels;
- and two longitudinal rods extending along the posterior part of the vertebral column on each side of the processus spinosus; each of these rods is fixed to the vertebral column at at least three points; the end attachment levels (top and bottom) must be formed of healthy elements of the skeleton, namely two vertebrae left intact, or one vertebra left intact for the bottom attachment level and the patient's occiput for the top level; and at least one other attachment level is formed from the above mentioned cage.

The invention will be better understood after reading the following description of preferred embodiments, given with reference to the following appended figures:
- Figure 1 that shows a side view of an example device according to the invention implanted on a patient's cervical vertebral column;
- Figure 2 that shows an anterior view of the same example device;
- Figure 3 that shows a sectional view of the same device, along line III-III in Figures 1 and 2;
- Figure 4 that shows a perspective view of an example of a cage used in the invention (Figure 4a) and an annular element forming the top and bottom ends (Figure 4b).

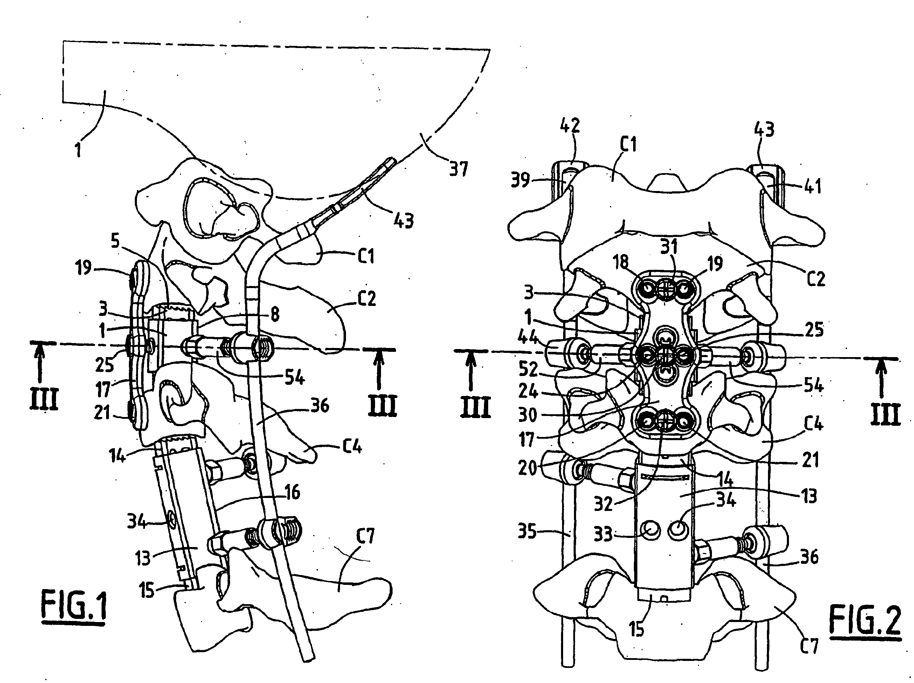

In the example that will be described and illustrated in detail in Figures 1 to 3, the bottom of the skull I of the patient and his cervical vertebral column have suffered from ablation of the vertebrae C3, C5 and C6 and the associated disks. Therefore, vertebrae C1, C2, C4 and C7 are kept intact.

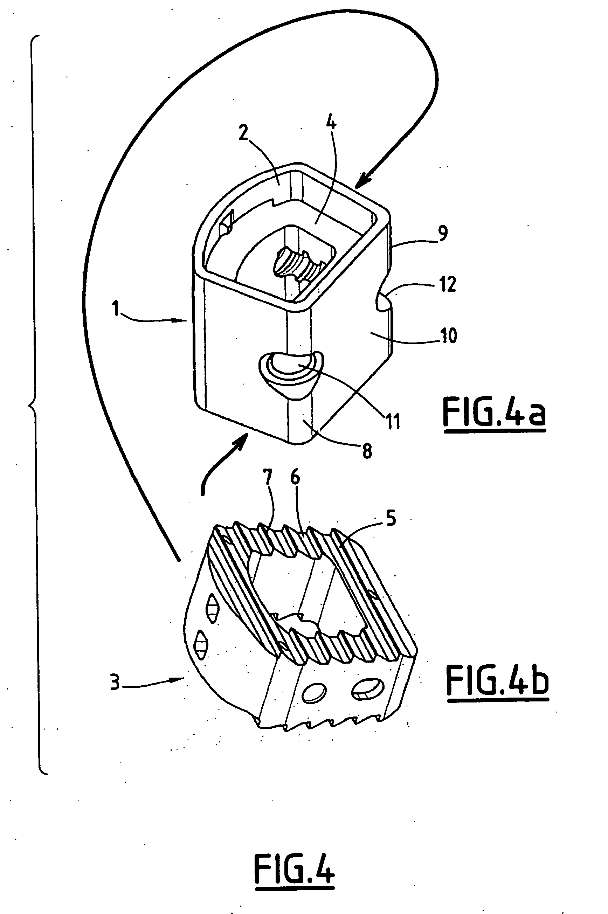

Vertebra C3 was replaced by a cage 1 (see Figure 4a) with a generally parallelepiped shape, defining a central compartment 2 in which a bone graft may be contained which, after placement of the device, will enable fusion between the vertebral plates of C2 and C4 facing each other. In the example shown, this cage 1 has annular elements 3 at both ends (see Figure 4a) that can be inserted and clipped inside the cage 1 (only one can be seen in Figures 1 and 2) to form the top and bottom surfaces of the cage 1. As can be seen in Figure 4a, the cage 1 comprises a central compartment 2 containing edges 4 on which the annular elements 3 can rest.

Each of these annular elements 3 makes contact between the cage 1 and the vertebral plate C2 or C4. To achieve this, they preferably have an etching at least around their periphery 5 with hollows. 6 and relief 7. The relief 7 must be embedded in the corresponding vertebral plate to improve the bond between the cage 1 and the vertebrae C2, C4.

Another function of these annular elements 3 is to apply the final configuration to the cage 1 in terms of length and angle of its end surfaces. As shown, the top and bottom surfaces of the annular element 3 might not be parallel. In this case, even if the cage 1 is approximately parallelepiped shaped as shown, it should be completed by annular elements 3 that form non-parallel top and bottom surfaces to restore the natural curvature to the vertebral column as closely as possible in the treated area.

Therefore, starting from a single basic model, it is possible to access different lengths of the cage 1 and angles of its end surfaces, simply by varying the shapes and dimensions of the annular elements used to complete the basic model.

The cage 1 has threaded orifices 11, 12 on the edges 8, 9 of its posterior face 10, and the function of these orifices will be described later.

Similarly, as can be seen in Figures 1 and 2, the vertebrae C5, C6 and their associated disks have been replaced by a cage 13 similar to cage 1, except that it is longer since it has to replace two vertebral bodies instead of one. Similarly, it is provided with two annular elements 14, 15 similar to the annular element 3. As a variant, the cage 13 may be replaced by a stack of two shorter cages nested around an annular element similar to elements 3, 14 and 15. Each posterior edge 16 of the cage 13 is provided with two threaded perforations distributed along its height, similar to the threaded perforations 11, 12 of cage 1.

The anterior plate 17 is also visible in Figures 1 to 3 and fixes the cage 1 to the vertebral bodies of C2 and C4. To achieve this, in the example shown, the plate 17 comprises two perforations close to each of its ends through which two screws 18, 19 pass penetrating into C2 and two screws 20, 21 pass penetrating into C4. Two orifices 22, 23 are formed in the central part of the plate 17, through which screws 24, 25 pass penetrating into corresponding threaded orifices 26, 27 of cage 1 to fix the plate 17 to cage 1. Preferably, the plate 17 comprises a housing 28 for a washer 29 fixed to the plate 17 by a screw 30. The periphery of the washer 29 bears on the heads of screws 24, 25 and prevents them from migrating outside their housings. Similar washers 31, 32 are also present to prevent screws 18, 19, 20, 21 fixing the plate 17 on C2 and C4 from migrating outside their corresponding housings.

A plate similar to plate 17 also optimises the bond or fusion between firstly the cage 13 replacing C5 and C6, and secondly C4 and C7. It is not shown in Figures 1 and 2, for better clarity. On the other hand, threaded orifices 33, 34 are shown that fix this plate on the cage 13 using screws.

The other elements stiffening the assembly' and its attachment to the patient's skeleton are two longitudinal rods 35, 36 that extend along the posterior side of the vertebral column on each side of the processus spinosus.

These rods 35, 36 must each be fixed at at least three levels onto the vertebral column. At least two of these attachment levels must be at healthy elements of the skeleton, namely two healthy vertebrae or one healthy vertebra and the patient's occiput 37. And the two rod ends (the highest and lowest on the vertebral column) must be attached to these healthy elements. The other attachment levels are ideally attachment points for all cages 1, 13 of the device according to the invention.

Figure 1 shows a case in which the rods 35, 36 are fixed to the occiput 37, on a level of the cage 1 replacing C3, on two levels of the cage 13 replacing C5 and C6 and on the vertebra C7. The rods 35, 36 are attached to the occiput 37 and C7 by screws not shown in Figure 1. The screws that fix the rods 35, 36 on the occiput 37 pass through the orifices 38, 39, 40, 41 formed for this purpose at the flattened top end 42, 43 of each rod 35, 36. The screws that fix the rods 35, 36 to one or more healthy vertebrae have a head in which a rod 35, 36 may be inserted and blocked, as is done conventionally, or a connector inside which the rod 35, 36 is blocked and that is itself fixed to the screw.

A preferred method of fixing the rods 35, 36 to the cages 1, 13 is shown in Figures 1 to 3, and will now be described with reference to the device that fixes the rod 35 extending on the right side of the vertebral column to the cage 1 that replaces C3.

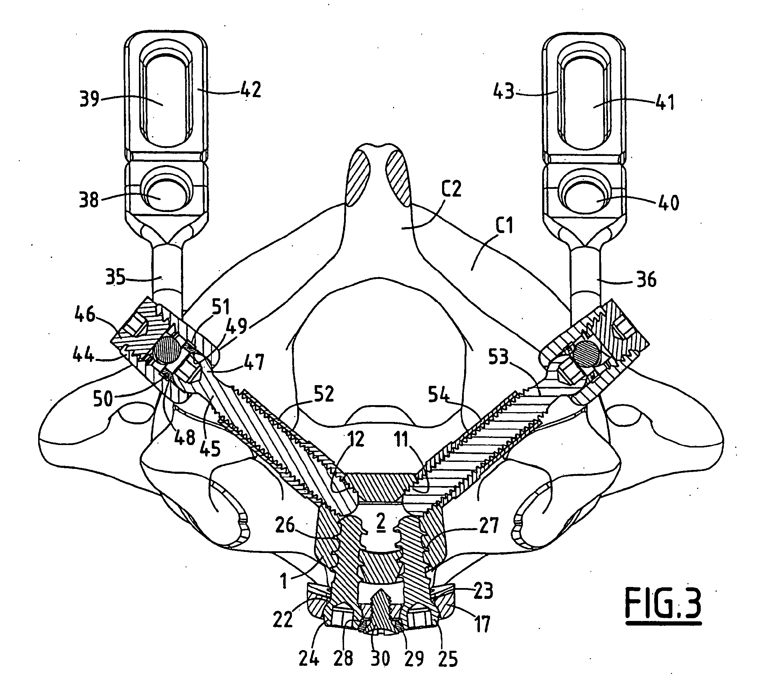

As can be seen better in Figure 3, the rod 35 is inserted into the tulip shaped head 44 of a screw 45, where it is blocked by a threaded plug 46, cooperating with the threaded inside walls of the head 44. In the example shown, the head 44 can be oriented multi-axially with respect to the longitudinal axis of the screw 45. Consequently, the screw 45 is provided with a spherical contact surface 47 with which the bottom part of the head 44 cooperates and an intermediate annular part 48 that has one face into which the rod 35 fits, and a spherical contact surface 49 on the other of its faces. An elastic ring 50 retains the annular part 47 in the head 44 in cooperation with a peripheral groove 51 formed on the internal face of the head 44. The multi-axially orientable nature of the head 44 allows the surgeon a great deal of freedom in placing the device as quickly as possible and making the device fit the anatomy and the needs of the patient in the best possible manner.

The screw 45 penetrates into the threaded orifice 12 formed on the edge 9 of the cage 1, at the end opposite the end at which the head 44 is located. Thus, due firstly to blocking of the rod 35 in the head 44 of the screw 45 achieved by the threaded plug 46, and secondly insertion of the screw 45 into the cage 1, a rigid link is achieved between the rod 35 and the cage 1.

Preferably, the screw 45 is not directly exposed to the outside medium over its entire length, but it passes through a sleeve 52 threaded on the inside and inserted on the cage 1 at the inlet of the orifice 12. Thus, any organs (spinal cord, nerve roots, etc.) that could come into contact with the thread of the screw 45 during and after placement of the device are protected from contact that could injure them. As a variant, this sleeve 52 could be screwed into the inlet of orifice 12 by means of an outer thread, instead of simply being inserted into it.

Symmetrically with the screw 45 - sleeve 52 assembly that has just been described, the cage 1 comprises a screw 53 - sleeve 54 assembly and its associated parts on its other edge 8, that similarly make the connection between the rod 36 extending on the left side of the vertebral column, with the cage 1.

Similarly, the cage 13 replacing C5 and C6 comprises screw - sleeve assemblies similar to the previous assemblies making the connection with the rods 35, 36. Only two are shown in Figures 1-3, but for reasons of symmetry and stiffness of the device, it would be preferable if there were four, namely two for each replaced vertebra.

Preferably, the orifices 11 and 12 provide an orientation for the screws 45, 53 corresponding approximately to the orientation with the natural processes, of the vertebra or vertebrae that the corresponding cage 1 is supposed to replace. In the example shown, this orientation forms an angle of 50° with the transverse axis of the vertebral column.

Obviously, variants of the device that has just been described may easily be imagined.

In particular, the connections of the different elements to each other and to the patient's skeleton may be different from those shown.

The presence of annular elements 3, 14 and 15 at the ends of cages 1, 13 is not compulsory, if it is acceptable to use single piece non-stackable cages. In this case, the relief 7 for attachment of the cage 1, 13 to the corresponding vertebral plates could be formed on the extreme edges of the cages 1, 13, the said edges also having the orientations required to restore the normal geometry of the vertebral column.

When, as shown, a device according to the invention comprises several cages 1, 13 separated by one or several healthy vertebrae it might be sufficient to only fix one of the cages to the healthy vertebrae that surround it using an anterior plate 17.

In order to place the device according to the invention, the surgeon begins by making an anterior corporectomy onto the vertebra to be replaced (for example C3). He then puts the cage 1 and its annular elements 3, if any, into place. He then puts the anterior plate 17 into place, firstly fixing it to the cage 1 and then to healthy vertebrae C2, C4. He then performs posterior surgery beginning by removing posterior bone elements (articular facets and processus articularis). He then fixes the screws 45 in the cage 1 and blocks them with the sleeves 52, 54. He then places posterior attachment devices of the rods 35, 36 on healthy parts of the skeleton. Finally, he puts rods 35, 36 into place, by making their connection with the screws 45 and posterior attachment devices.

The device according to the invention is particularly well adapted for use in the cervical region. In this region, it can advantageously be used instead of known devices that require both anterior and posterior stabilisation. However it can be used in other regions of the vertebral column.

## Claims

1. A vertebral column osteosynthesis device of the type comprising at least one cage (1, 13) containing a bone graft that will be substituted for at least one vertebra, and the vertebrae (C2, C4; C7), between which the cage (1, 13) is placed, are fused with at least one anterior plate (17) that will be fixed to the said vertebrae (C2, C4) between which the said cage (1, 13) or at least one of the said cages (1, 13) is placed, **characterised in that** the said device also comprises:
- means (24, 25) of attachment of the said anterior plate (17) or at least one of the said anterior plates (17) to the cage (1) facing it;
- two longitudinal rods (35, 36) that will extend along the vertebral column on each side of the processus spinosus, means of attachment of the ends of the said rods to healthy elements of the patient's skeleton, and means of connection of each rod to the said cage (1, 13) or to at least one of the said cages (1, 13).

2. Device according to claim 1, **characterised in that** the said cage (1, 13) comprises an annular element (3, 4) at at least one of its ends for stacking two cages (1, 13) or for anchoring the said cage (1, 13) in a vertebral plate.

3. Device according to claim 1 or 2, **characterised in that** the said means of connection of each rod (35, 36) to the said cage (1, 13) are composed of threaded orifices (11, 12) formed in the cage (1,13) and screws (45, 53) penetrating into the said threaded orifices (11, 12), the heads of each of the said screws (45, 53) comprising means of reception (44) and locking (46) of one of the said rods (35, 36).

4. Device according to claim 3, **characterised in that** the said threaded orifices (11, 12) are formed on posterior edges (8, 9) of the cage (1, 13) and control the orientations of the said screws (45, 53) making them correspond to the orientations of the natural processes of the vertebrae to be replaced by the cage (1, 13).

5. Device according to claim 3 or 4, **characterised in that** the said screws (45, 53) may be inserted into sleeves (52, 54) connected to the said cage (1, 13).

6. Device according to one of claims 1 to 5, **characterised in that** one of the ends (42, 43) of the said rods (35, 36) are shaped so that they can be fixed to the patient's occiput (37).

7. Device according to one of claims 1 to 6, **characterised in that** it comprises several cages (1 to 13), and one anterior plate (17) for each cage.

8. Device according to one of claims 1 to 7, **characterised in that** at least one of the said cages (13) comprises means of connection to the said rods (35, 36) distributed on several of its levels.

9. Device according to one of claims 1 to 8, **characterised in that** the dimensions and shape of the said at least one cage (1, 13), possibly provided with one or several annular elements (3, 14, 15), are compatible with the replacement of a cervical vertebra or several successive cervical vertebrae.

## Patentansprüche

1. Wirbelsäulen-Osteosynthesevorrichtung des Typs mit zumindest einem Käfig (1, 13), der ein Knochenimplantat bzw. einen Knochenspan enthält, das bzw. der zumindest einen Wirbel ersetzen wird, und bei dem die Wirbel (C2, C4, C7), zwischen die der Käfig (1, 13) plaziert ist bzw. wird, durch wenigstens eine anteriore Platte (17) fusioniert bzw. zusammengefasst werden, die an die Wirbel (C2, C4) fixiert werden wird, zwischen die der Käfig (1, 13) oder wenigstens einer der Käfige (1, 13) plaziert ist, **dadurch gekennzeichnet, dass** die das die Vorrichtung auch aufweist:
- Mittel (24, 25) der Anbringung der anterioren Platte (17) oder wenigstens einer der anterioren Platten (17) an den Käfig (1), der ihr gegenüberlegt,
- zwei Längsstäbe (35, 36), die sich entlang der Wirbelsäule auf jeder Seite des Prozessus spinosus bzw. Dornfortsatzes erstrecken werden, Mitteln der Anbringung der Enden der Stäbe an gesunde Elemente des Skeletts des Patienten, und Mittel der Verbindung jedes Stabs mit dem Käfig (1,13) oder wenigstens einem der Käfige (1, 13).

2. Vorrichtung nach Anspruch 1, **dadurch gekennzeichnet, dass** der Käfig (1, 13) an wenigstens einem seiner Enden ein ringförmiges Element (3, 4) aufweist, um zwei Käfige (1, 13) aufeinander zu stapeln, oder um den Käfig (1, 13) in bzw. an einer Wirbelplatte zu verankern.

3. Vorrichtung nach Anspruch 1 oder 2, **dadurch gekennzeichnet, dass** die Mittel zur Verbindung jedes Stabes (35, 36) mit dem Käfig (1, 13) aus mit Gewinden versehenen Öffnungen (11, 12), die im Käfig ausgebildet sind (1, 13), und Schrauben (45, 53), die in die mit Gewinden versehenen Öffnungen eindringen (11, 12), gebildet sind, wobei die Köpfe jeder der Schrauben (45, 53) Mittel der Aufnahme (44) und des verriegelns (46) eines der Stäbe (35, 36) aufweisen.

4. Vorrichtung nach Anspruch 3, **dadurch gekennzeichnet, dass** die mit Gewinden versehenen Öffnungen (11, 12) an posterioren Kanten (8, 9) des Käfigs (1, 13) ausgebildet sind und die Ausrichtung der Schrauben (45, 53) kontrollieren und bewerkstelligen, dass jene den Orientierungen der natürlichen Fortsätze der Wirbel, die durch den Käfig (1, 13) ersetzt werden sollen, entsprechen.

5. Vorrichtung nach Anspruch 3 oder 4, **dadurch gekennzeichnet, dass** die Schrauben (45, 53) in Muffen bzw. Hülsen (52, 54), die mit dem Käfig (1, 13) verbunden sind, eingeführt werden können.

6. Vorrichtung nach einem der Ansprüche 1 bis 5, **dadurch gekennzeichnet, dass** eines der Enden (42, 43) der Stäbe (35, 36) so geformt sind, dass sie an das Hinterhaupt bzw. das Occiput (37) des Patienten fixiert werden können.

7. Vorrichtung nach einem der Ansprüche 1 bis 6, **dadurch gekennzeichnet, dass** sie mehrere Käfige (1 bis 13) und eine anteriore Platte (17) für jeden Käfig aufweist.

8. Vorrichtung nach einem der Ansprüche 1 bis 7, **dadurch gekennzeichnet, dass** wenigstens einer der Käfige (13) Mittel zur Verbindung der Stäbe (35, 36) aufweist, die auf mehreren seiner Ebenen verteilt sind.

9. Vorrichtung nach einem der Ansprüche 1 bis 8, **dadurch gekennzeichnet, dass** die Abmessungen und die Form des zumindest einen Käfigs (1, 13), der möglicherweise mit einem oder mehreren ringförmigen Elementen (3, 14, 15) ausgebildet ist, kompatibel mit der Ersetzung eines zervikalen Wirbels oder mehrerer aufeinanderfolgender zervikaler Wirbel sind.

## Revendications

1. Dispositif d'ostéosynthèse pour colonne vertébrale, du type comprenant au moins une cage (1, 13) contenant une greffe osseuse qui sera substituée à au moins une vertèbre, et les vertèbres (C2, C4, C7) entre lesquelles la cage (1, 13) est placée sont fusionnées avec au moins une plaque antérieure (17) qui sera fixée auxdites vertèbres (C2, C4) entre lesquelles ladite cage (1, 13) ou l'une au moins desdites cages (1, 13) est placée, **caractérisé en ce que** ledit dispositif comprend également :
- des moyens (24, 25) pour attacher ladite plaque antérieure (17) ou l'une au moins desdites plaques antérieures (17) à la cage (1) qui lui fait face;
- deux tiges longitudinales (35, 36) qui vont s'étendre le long de la colonne vertébrale sur chaque côté du processus épineux, des moyens pour attacher les extrémités desdites tiges à des éléments sains du squelette du patient, et des moyens pour connecter chaque tige à ladite cage (1, 13), ou à l'une au moins desdites cages (1, 13).

2. Dispositif selon la revendication 1, **caractérisé en ce que** ladite cage (1, 13) comprend un élément annulaire (3, 4) à l'une au moins de ses extrémités pour empiler deux cages (1, 13) ou pour ancrer ladite cage (1, 13) dans une plaque vertébrale.

3. Dispositif selon la revendication 1 ou 2, **caractérisé en ce que** lesdits moyens de connexion de chaque tige (35, 36) à ladite cage (1, 13) sont composés d'orifices taraudés (11, 12) formés dans la cage (1, 13) et de vis (45, 53) qui pénètrent dans lesdits orifices taraudés (11, 12), les têtes de chacune desdites vis (45, 53) comprenant des moyens de réception (44) et de blocage (46) de l'une desdites tiges (35, 36).

4. Dispositif selon la revendication 3, **caractérisé en ce que** lesdits orifices taraudés (11, 12) sont formés sur des bords postérieurs (8, 9) de la cage (1, 13) et contrôlent les orientations desdites vis (45, 53) en les amenant à correspondre aux orientations des processus naturels des vertèbres à remplacer par la cage (1, 13).

5. Dispositif selon la revendication 3 ou 4, **caractérisé en ce que** lesdites vis (45, 53) peuvent être insérées dans des manchons (52, 54) connectés à ladite cage (1, 13).

6. Dispositif selon l'une des revendications 1 à 5, **caractérisé en ce qu'**à l'une des extrémités (42, 43) lesdites tiges (35, 36) sont conformées de telle façon qu'elles peuvent être fixées à l'occiput du patient (37).

7. Dispositif selon l'une des revendications 1 à 6, **caractérisé en ce qu'**il comprend plusieurs cages (1 à 13) et une plaque antérieure (17) pour chaque cage.

8. Dispositif selon l'une des revendications 1 à 7, **caractérisé en ce que** l'une au moins desdites cages (13) comprend des moyens de connexion auxdites tiges (35, 36) distribués sur plusieurs de ses niveaux.

9. Dispositif selon l'une des revendications 1 à 8, **caractérisé en ce que** les dimensions et la forme de ladite au moins une cage (1, 13), éventuellement dotée d'un ou plusieurs éléments annulaires (3, 14, 15), sont compatibles avec le remplacement d'une vertèbre cervicale ou de plusieurs vertèbres cervicales successives.
